# EUROPEAN PATENT SPECIFICATION

(11) **EP 1 222 942 B1**
(45) Date of publication and mention of the grant of the patent: **21.06.2006**
(21) Application number: 02250262.9
(22) Date of filing: 15.01.2002
(51) Int. Cl.: A61N 1/37

(54) **System for automating capture verification assessment and pacing threshold assessment using a programmer**
Vorrichtung zur Festsetzung des Einfangens und des Schwellwertes mittels einer Programmiereinrichtung
Système pour la vérification de capture et du seuil utilisant un programmateur

(30) Priority: 16.01.2001 US 764617
(43) Date of publication of application: 17.07.2002
(73) Proprietor: PACESETTER, INC., Sylmar, CA 91392-9221 (US)
(72) Inventor: Bornzin, Gene A, Simi Valley, CA 93065 (US); Valikai, Kenneth, Palos Verdes Pen., CA 90274 (US); Florio, Joseph J., La Canada, CA 91011 (US); Snell, Jeffrey D., Chatsworth, CA 91311 (US)
(74) Representative: Rees, David Christopher

(56) References cited:
- US-A- 4 055 189
- US-A- 5 702 427
- US-A- 5 871 512

## Description

The present invention relates in general to implantable cardiac stimulation devices, including bradycardia and anti-tachycardia implantable stimulation devices, defibrillators, cardioverters and combinations thereof that are capable of measuring, storing, and transmitting physiological data and parametric data pertaining to implantable medical devices. More particularly, this invention relates to a system for automating the threshold assessment process by utilizing a programmer device with specialized software for the purpose of assessing capture verification in conjunction with an implantable cardiac stimulation device.

Implantable medical devices, such as implantable stimulation devices, defibrillators, and cardioverters (collectively referred to as implantable cardiac stimulating devices) are designed to monitor and stimulate the heart of a patient that suffers from a cardiac arrhythmia. Using leads in contact with a patient's heart, these devices typically stimulate the cardiac muscles by delivering electrical pulses in response to detection of cardiac events, which are indicative of a cardiac arrhythmia. Properly administered therapeutic electrical pulses often successfully reestablish or maintain the heart's regular rhythm.

Implantable cardiac stimulating devices can treat a wide range of cardiac arrhythmias by using a series of adjustable parameters to alter the stimulus energy, the shape, the location, and the frequency of the therapeutic pulses. The adjustable parameters are usually defined in a computer program stored in a memory of the implantable device. The program (which is responsible for the operation of the implantable device) can be defined or altered telemetrically by a medical practitioner using an implantable device programmer. Modern implantable devices have a great number of adjustable parameters that must be tailored to a particular patient's therapeutic needs.

One adjustable parameter of particular importance in implantable stimulation devices, is an implantable stimulation device's stimulus energy, which is a function of the stimulus pulse amplitude and pulse width. "Capture" is defined as a cardiac response to an implantable stimulation device stimulation pulse. When an implantable stimulation device stimulation pulse stimulates either a heart atrium or a heart ventricle during an appropriate portion of a cardiac cycle, it is desirable to have the heart properly respond to the stimulus provided. Every patient has a "capture threshold" which is generally defined as a minimum amount of stimulation energy to effect capture. Capture should be achieved at the lowest possible energy setting yet provide enough of a safety margin so that should a patient's threshold increase, the output of an implantable stimulation device (i.e. the pacing stimulus energy) would be sufficient to maintain capture. Dual-chamber implantable stimulation devices may have differing atrial and ventricular pacing thresholds that correspond to atrial and ventricular capture thresholds, respectively.

The earliest implantable stimulation devices had a predetermined and unchangeable pacing stimulus energy, which proved to be problematic because the capture threshold is not a static value. The capture threshold may be affected by a variety of physiological and other factors. For example, certain cardiac medications may temporarily raise or lower the capture threshold from its normal value. In another example, fibrous tissue that forms around implantable stimulation device lead tips within several weeks after implantation may raise the capture threshold. As a result, some patients eventually suffered from loss of capture as their implantable stimulation devices were unable to adjust the pre-set pacing stimulus energies to overcome the changed capture thresholds. One solution was to set the level of stimulation energy fairly high so as to provide sufficient safety margin and avoid loss of capture due to a change in the capture threshold. However, this approach resulted in some discomfort from skeletal muscle stimulation in patients who had to endure high levels of cardiac stimulation energy. Furthermore, such stimulation pulses consumed extra battery resources, thus shortening the useful life of an implantable stimulation device.

When programmable implantable stimulation devices were developed, the pacing stimulus energy was implemented as an adjustable parameter that could be set or changed by a medical practitioner. Typically, such adjustments were made by the medical practitioner using an external programmer capable of communication with an implanted implantable stimulation device via telemetry through a programming wand applied to a patient's chest. Pulsed magnetic fields applied over the implantable stimulation device were also used to modify pacing parameters. The particular setting for the implantable stimulation device's stimulus energy was usually derived from results of extensive physiological tests performed by the medical practitioner to determine the patient's capture threshold, from the patient's medical history, and from the patient's list of medications. Thus, the stimulus energy setting required consideration of the capture threshold and safety margin. While the adjustable pacing stimulus energy feature proved to be superior to the previously known static stimulus energy, some significant problems remained unsolved. In particular, when a patient's capture threshold changed, the patient was forced to visit the medical practitioner to adjust the pacing stimulus energy accordingly.

To address these needs, implantable stimulation device manufacturers have developed advanced implantable stimulation devices that are capable of determining a patient's capture threshold and automatically adjusting the stimulation pulses to a level just above that which is needed to maintain capture. This approach, referred herein as "autocapture", improves the patient's comfort, reduces the necessity of unscheduled visits to the medical practitioner, improves patient safety, and greatly increases the implantable stimulation device's battery life by conserving the energy used for stimulation pulses. Additionally, such an implantable stimulation device maintains a record of each threshold assessment and the resulting stimulus energy required to maintain capture. This record keeping by the implantable stimulation device is of benefit to the medical practitioner in that it provides a record of lead stability and chronic performance.

These and other advanced implantable stimulation device systems utilize a variety of robust sensing techniques to verify that capture takes place after a stimulating pulse is delivered to the heart. For example, capture may be verified by analyzing cardiac signals and comparing them to a polarization template and then determining the presence of an evoked response, as is disclosed in commonly assigned U.S. Patent No. 5,417,718 (Kleks et al.).

Alternately, capture assessment may take place by assessing mechanical changes in the heart associated with a contraction, such as the motion of a cardiac wall or by direct detection and measurement of the cardiac evoked response immediately following a pacing pulse, as is disclosed in commonly assigned U.S. Patent No. 5,549,652 (McClure et al.

Another such advanced implantable stimulation device system is disclosed in commonly assigned U.S. Pat. No. 4,817,605 (Sholder), in which capture is verified by monitoring time intervals between atrial stimulation pulses and P-wave occurrences.

Regardless of the exact method used to determine threshold, the pacing stimulus energy is then automatically set at a level just above that necessary to maintain capture. As the patient's capture threshold changes, the pacing stimulus energy is correspondingly automatically adjusted.

After initial implantation and configuration of the implantable stimulation device, the medical practitioner typically performs periodic follow-up examinations to determine if the therapy delivered by the device is having the desired effect and the implantable stimulation device is otherwise operating properly. In particular, it is of utmost importance to verify that the implantable stimulation device's pacing stimulus energy is sufficient to maintain capture with an adequate safety margin to consider changes in capture threshold. As was previously discussed, the capture threshold may change over time as a result of a variety of factors, such as fibrous tissue growth on implantable stimulation device lead tips or the regimen of the patient's medication. Such changes in the capture threshold may have caused loss of capture in an older implantable stimulation device model. Thus, the medical practitioner must determine whether capture is present, whether there is sufficient stimulus safety margin, and when capture is not present, adjust the pacing stimulus energy to reestablish capture.

To assist the medical practitioner, semi-automatic pacing threshold determination procedures have been developed in recent years. In a typical semi-automatic procedure the medical practitioner applies surface electrocardiogram (ECG) electrodes to the patient and then configures the ECG system for capture testing. Using the electrocardiograph as a monitor of pacing function, the medical practitioner observes implantable stimulation device performance and whether capture is occurring. When capture is observed, the medical practitioner uses a programmer that communicates with the implantable stimulation device, and initiates a semi-automatic capture test while observing the implantable stimulation device's operation. The semi-automatic capture test sequentially decrements the stimulus energy while the medical practitioner determines that capture is taking place. In this way, the medical practitioner provides capture verification. As soon as loss of capture is observed the medical practitioner terminates the test and records the stimulus energy where capture was maintained just prior to the step where capture was lost. The final pacing stimulus energy is then adjusted by setting the output level sufficiently high to effect capture and maintain an adequate safety margin.

However, such a procedure involving semi-automatic pacing threshold assessment and observation review for capture verification is a time consuming and complex task requiring significant attention and effort on the part of the medical practitioner. The placing and subsequent removal of ECG electrodes is an intensive and time consuming task. In addition, the medical practitioner must spend a significant amount of time configuring the ECG system for the patient's individual characteristics. The medical practitioner must also manually examine the ECG readout and analyze the cardiac waveform to determine whether capture is present both during the initial capture threshold assessment and during subsequent follow-up. Finally, while many follow-up tests of advanced implantable stimulation devices may be performed remotely via modem or other communication device, the patient must visit the medical practitioner's office for the pacing threshold determination and capture verification procedure.

It would thus be desirable to provide a system for fully automating the pacing threshold determination and capture verification procedure. It Would be desirable that the results of the capture threshold assessment be automatically documented and the programmer automatically providers a recommended setting for the pacing stimulus energy. It would also be desirable to provide the medical practitioner with the ability to perform the procedure without the use of intensive time consuming devices such as a surface ECG. It would further be desirable to provide the medical practitioner with the ability to perform the procedure on a patient remotely.

US 5,702,427 discloses a capture verification system for a cardiac pacemaker which employs a pressure wave sensor mounted in the pulse generator for sensing pressure waves transmitted from the distal end of the pacing need. The pressure waves include characteristic sounds of heart contraction and/or distal end lead emotion caused by the contraction motion of the patient's heart. A further, isolated, reference sensor is also incorporated into the pulse generator in a similar fashion.

The disadvantages and limitations discussed above are overcome by the present invention. In accordance with the invention, a system that is initiated by a medical practitioner, is provided for automating the pacing threshold assessment procedure and capture verification by the stimulation device or by the programmer of proper capture by stimulus pulses from a patient's implantable cardiac stimulation device, and to automatically adjust the device's pacing stimulus energy if necessary. The invention is defined in

claim 1. The System of the present invention also utilizes information processing, markers & other annotation and output capabilities of an implantable device programmer to enable the medical practitioner to observe the automatic procedure and to verify that it is performed properly.

The programmer may also be used to initiate and observe the pacing threshold assessment with automatic capture verification procedure for implantable stimulation devices that do not inherently by design possess the self-determinant capability of morphology detection to determine capture.

The programmer may also be used to remotely initiate and assess capture verification and provide a threshold assessment when the patient is at a different geographic location than the medical practitioner. The system of this invention may also automatically document the capture threshold and make recommendations for adjusting the proper stimulus energy level. All of the aforementioned advantages and features are achieved without incurring any substantial relative disadvantage.

An implantable cardiac stimulation device equipped with data acquisition and telemetric communication capabilities, and also provides an implantable device programmer, preferably in the form of a portable computer, with data processing, data storage, graphical data display, data output, data communication, telemetric communication, and diagnostic capabilities are provided.

The implantable stimulation device includes a control system for controlling the operation of the implantable stimulation device, a connector adapted to couple to a set of leads for receiving atrial and ventricular signals and for delivering atrial and ventricular stimulation pulses, a set of amplifiers for amplifying the atrial and ventricular signals, and pulse generators for generating atrial and ventricular stimulation pulses. In addition, the implantable stimulation device includes memory for storing operational parameters for the control system, such as the value for stimulus pacing energy to affect capture, and for storing data acquired by the control system for later retrieval by the medical practitioner using an external programmer. The device also includes a telemetry circuit for communicating with the external programmer. The implantable stimulation device may also indude an optional sensor for sensing mechanical changes within the heart.

The programmer includes a control system with specialized software for controlling the operation of the programmer and for analyzing data acquired from the implantable stimulation device, a user input device for enabling the medical practitioner to issue commands to the programmer and to the implantable stimulation device, and an output device such as a video display for displaying data and images to the medical practitioner. The programmer also includes a memory for storing data and programs that perform various programmer functions and procedures, and a data acquisition device, such as a telemetry wand, for communicating with the implantable stimulation devices. A printer may optionally be connected to the programmer to provide a printed copy of the programmer's output. Finally, a remote communication device, such as a modem, may be connected between the telemetry circuit of the implantable stimulation device and the data acquisition device of the programmer to enable remote communication there between.

The medical practitioner may use the programmer to establish communication with the implantable stimulation device, selects a particular chamber of the patient's heart representing the tissue interface to be evaluated and initiate an automatic capture verification assessment in the selected chamber.

Optionally, if the programmer and/or the implantable stimulation device are equipped with more than one type of automatic capture assessment procedure, the medical practitioner may select a particular type of capture assessment (e.g., threshold assessment, capture verification at the current settings, etc.).

When selecting, for example, capture verification, the programmer/implantable stimulation device system then performs the capture verification assessment and composes a test record display based on, preferably, an intracardiac electrogram (IEGM), or optionally on a surface ECG, or on another type of time-based diagram representative of the cardiac events (e.g., a simulation of the surface ECG using IEGM signals). The various events, such as pacing pulses and sensed events, are then automatically marked with appropriately configured markers. In particular, the presence or absence of capture after pacing pulses are delivered is noted on the test record in appropriate locations. Optionally, the duration and amplitude of the pacing pulses may also be recorded on the test record. The test record is then displayed to the medical practitioner. The medical practitioner can then analyze the test record to verify whether the pacing stimulus energy is adequate and cardiac capture is appropriate.

When desired, the medical practitioner may also initiate an fully automatic pacing threshold assessment test. While the threshold assessment test is performed by the programmer and/or the implantable stimulation device, a fully annotated threshold assessment record is generated and displayed to the medical practitioner for review and analysis. The threshold assessment will proceed automatically to adjust the amplitude and/or pulse width until capture is lost. Upon detection of loss of capture, the pacing energy will return to pretest values and the results of the assessment will be displayed. The programmer, with specialized software may make a recommendation for the setting of the pacing stimulus energy based upon the type and model of implantable stimulation device system being evaluated. The embodied programmer algorithm may also make allowances for other data such as remaining battery capacity, medication regime, and the patient's degree of dependency on the implantable stimulation device.

Alternatively the capture verification assessment and subsequent threshold assessment procedure may be fully automatic such that the programmer and/or the implantable stimulation device system will initiate the complete test, stop automatically after the capture threshold is found, return to the recommended values determined by the device or programmer, and then display capture verification recordings at the recommended settings.

Also, if for some reason the test can not be completed automatically, the programmer will alert the medical practitioner after the test procedure is initiated as to why the test can not proceed automatically and what data is required to continue the assessment procedure.

It shoilild be understood that as a natterof design choice, the threshold assessment and automatic capture verification procedure may be performed entirety at the implantable stimulation device, entirely at the programmer, or both at the implantable simulation device and at the programmer without departing from the sprit of the invention

The system of the present invention thus greatly assists the medical practitioner in verifying capture and in determining a proper pacing threshold as a function of the patient's implantable stimulation device system, by fully automating assessment of the capture verification and pacing threshold assessment procedure such that the medical practitioner only needs to verify the automatic procedure's results. Furthermore, the present invention enables the procedure to be performed remotely.

The invention provides a system for automating review of capture verification by a medical practitioner, the system being configured for use with an implantable stimulation device implanted in a patient and a programmer operated by the medical practitioner and configured to remotely communicate with the implantable stimulation device, the system comprising: autocapture means for performing automatic capture verification through the implantable stimulation device to detect a presence of a captured cardiac event and an absence of a captured cardiac event when the captured cardiac event is expected; control means for generating a visual representation; and display means for displaying the visual representation to the medical practitioner to permit the medical practitioner to examine and analyse the performance of the automatic capture verificationcharacterised in that the visual representation is a representation of the presence and absence of the captured cardiac event and the control means further comprises means for making the captured cardiac event in the visual representation with as visual marker representative of capture and means for marking absence of the captured cardiac event with a visual marker representative of absence of capture in a location in the visual representation where the captured cardiac event was expected to occur.

Preferably, the step of performing automatic capture verification through one of the implantable stimulation device or the programmer, further comprises the steps of obtaining an intracardiac electrogram through the implantable stimulation device and performing the automatic capture verification using the intracardiac electrogram.

Preferably, the step of performing automatic capture verification through one of the implantable stimulation device or the programmer, further comprises the steps of obtaining an surface electrocardiogram through the programmer and performing the automatic capture verification using the surface electrocardiogram.

The method may further include recording, in the visual representation, the amplitude and duration characteristics for each pacing event. It may also comprise the steps of marking the captured cardiac event in the visual representation with a visual marker representative of capture and marking the absence of the captured cardiac event with a visual marker representative of absence of capture in a location in the visual representation at which the captured cardiac event was expected to occur. It may also comprise the steps of automatically assessing a pacing threshold of the implantable stimulation device, determining a recommended pulse amplitude and pulse width above the pacing threshold to ensure capture, and displaying the recommended pulse amplitude and pulse width on the programmer.

The invention enables a method for automating review of capture verification by a medical practitioner, the method being implemented in an implantable stimulation device implanted in a patient and a programmer operated by the medical practitioner and configured to remotely communicate with the implantable stimulation device, the method comprising the steps of:
(a) selectively initiating automatic capture verification by the medical practitioner;
(b) performing automatic capture verification through the implantable stimulation device;
(c) detecting, by at least one of the implantable stimulation device and the programmer, presence of cardiac events and absence of expected cardiac events during the automatic capture verification;
(d) identifying, by at least one of the implantable stimulation device and the programmer, a captured cardiac event when the captured cardiac event is detected, and when an expected capture event is not detected, identifying an absence of the expected captured cardiac event;
(e) marking, by at least one of the implantable stimulation device and the programmer, at least one of the identified captured cardiac event and the absence of the expected captured cardiac event with a pre-determined corresponding event marker
(f) generating, by at least one of the implantable stimulation device and the programmer, a visual representation of the identified cardiac events and the event markers; and
(g) displaying, at the programmer, the visual representation to the medical practitioner to permit the medical practitioner to examine and analyse the performance of the automatic capture verification.

The above and further features, advantages and benefits of the invention will become apparent in the following description taken in conjunction with the following drawings. It is to be understood that the foregoing general description and the following detailed description are exemplary and explanatory but are not to be restrictive of the invention.

The accompanying drawings illustrate one of the embodiments of the invention and, together with the description, serve to explain the principles of the invention in general terms. Like numerals refer to like parts throughout the disclosure.
**FIG.1** is a block diagram of a dual-chamber implantable stimulation device and a programmer in accordance with the principles of the present invention;
**FIG. 2** is a logic flow diagram representing an automatic capture verification and pacing threshold determination control program executed by one or both of the control system of the implantable stimulation device of **FIG. 1**, and the programmer control system of the programmer of **FIG**. **1**, in accordance with the principles of the present invention;
**FIG. 3** depicts an exemplary atrial capture verification and threshold assessment record output of the control program of **FIG. 2** in accordance with the principles of the present invention; and
**FIG. 4** depicts an exemplary ventricular capture verification and threshold assessment record output of the control program of **FIG. 2** in accordance with the principles of the present invention.

The system of the present invention makes use of an implantable stimulation device and an external programmer operated by a medical practitioner to perform an automatic capture verification and pacing threshold assessment test and to display the results of the test to the medical practitioner for review and analysis thereof.

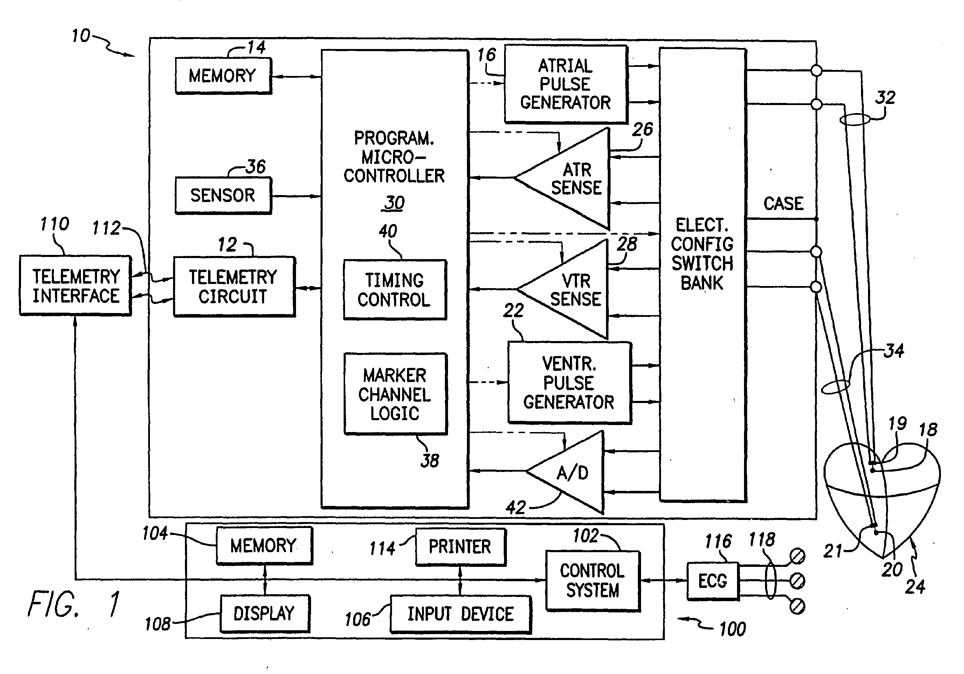

An implantable stimulation device 10 in accordance with this invention is shown in **FIG. 1**. The implantable stimulation device 10 is coupled to a heart 24 by way of leads 32 and 34, the lead 32 having a tip and ring electrodes 18, 19 which are in contact with one of the atria of the heart 24, and the lead 34 having a tip and ring electrode 20, 21 which are in contact with one of the ventricles. The lead 32 delivers stimulating pulses from an atrial pulse generator 16 to the atrium, while the lead 34 delivers stimulating pulses from a ventricular pulse generator 22 to the ventricle. In addition, electrical signals from the atria are delivered by the lead 32 to the input terminal of an atrial sense amplifier 26. Electrical signals from the ventricles are delivered through the lead 34 to the input terminal of a ventricular sense amplifier 28.

Controlling the dual-chamber implantable stimulation device 10 is a control system 30. The control system 30 is preferably a microprocessor-based system such as the one disclosed in commonly assigned U.S. Patent No. 4,940,052 of Mann. The control system 30 may also be a state logic-based system such as the one disclosed in U.S. Patent No. 4,944,298 of Sholder. The control system 30 also includes a real-time clock (not shown) for providing timing for monitoring cardiac events and for timing the application of therapeutic pulses by the pulse generators 16 and 22. Finally, the control system 30 may optionally include circuitry (not shown) for verifying capture and determining the pacing threshold, such for example as disclosed in the U.S. Patent No. 5,417,718 to Kleks et al.; U.S. Patent No. 5,549,652 to McClure et al.; and U.S. Patent No. 4,817,605 to Sholder.

The implantable stimulation device 10 also includes a memory 14 which is coupled to the control system 30. The memory 14 allows certain control parameters used by the control system 30 in controlling the operation of the implantable stimulation device 10 to be telemetrically stored and modified, as required, in order to customize the operation of the implantable stimulation device 10 to suit the needs of a particular patient. In particular, the pacing capture threshold parameters for the atrial and ventricular pacing pulses are stored in the memory 14. In addition, data sensed during the operation of the implantable stimulation device 10 may be stored in the memory 14 for later retrieval and analysis.

The control system 30 receives the output signals from the atrial amplifier 26 and from the ventricular amplifier 28 each time that an atrial event (e.g., a P-wave) or a ventricular event (e.g., an R-wave), respectively, are sensed within the heart 24.

An A/D converter 42 is shown for providing the intracardiac electrograms (IEGM's)from the leads 32 and 34 to the control system 30. Based on the IEGM's, the controller 30 can apply markers for sensed and paced events, and timing of events, using a marker channel logic unit 38 and a timing and control unit 40, either internal to the control system software/hardware (as shown) or external.

The control system 30 also generates an atrial trigger signal which is sent to the atrial pulse generator 16, and a ventricular trigger signal which is sent to the ventricular pulse generator 22. These trigger signals are generated each time that a stimulation pulse is to be generated by one of the pulse generators 16 or 22. The atrial stimulation pulse is referred to simply as the "A-pulse," and the ventricular stimulation pulse is referred to as the "V-pulse". The characteristics of these stimulation pulses are determined by the pacing stimulus energy settings that are stored in the memory 14.

During the time that either an A-pulse or a V-pulse is being delivered to the heart 24, the corresponding atrial amplifier 26 or the ventricular amplifier 28 is typically disabled by way of a blanking signal presented to the appropriate amplifier 26 or 28 from the control system 30. This blanking action prevents the amplifiers 26 and 28 from becoming saturated with the relatively large stimulation pulses that are present at their input terminals during pacing pulse delivery. This blanking action also prevents residual electrical signals (known as "after-potentials" or polarization) present in the muscle tissue as a result of the implantable stimulation device stimulation from being interpreted as atrial or ventricular events.

The implantable stimulation device 10 may include an optional sensor 36 connected to the control system 30 for providing additional capture verification by sensing mechanical changes associated with the contraction of the heart 24 and/or for rate modulation. Examples of a suitable sensor 36 include, but are not limited to, a cardiac wall motion sensor, a cardiac accelerometer, an integral pressure transducer, or an electrical impedance-based ventricular volume sensor. The sensor 36 may also be disposed on one or both of the leads 32 and 34, respectively.

A telemetry circuit 12 is further included in the implantable stimulation device 10 connected to the control system 30. The telemetry circuit 12 may be selectively coupled to an external programmer 100 by means of an appropriate communication link 112. The communication link 112 may be an electromagnetic telemetry link or a remote communication link such as a pair of modems interconnected via a telecommunications link and equipped with telemetry capabilities.

The programmer 100 is controlled by a programmer control system 102, which is preferably microprocessor-based. A programmer memory 104 is used by the programmer control system 102 for software operation, data processing, and long-term data storage. The programmer memory 104 may include random access memory and any type of memory suitable for long-term data storage including a hard disk drive, flash memory, or a rewritable optical disk. Optionally, one or more capture verification assessment and threshold assessment programs may be stored in the programmer memory 102 for selective use by the medical practitioner.

The programmer 100 is also provided with an display device 108. The display device 108 is used to display results of a capture verification test obtained from the implantable stimulation device 10 or performed by the programmer 100. An telemetry interface 110 is used to communicate with the implantable stimulation device 10 via the communication link 112. The telemetry interface 110 may be a telemetry wand or another type of communication device, for wireless communication with the implantable stimulation device 10.

If desired, the programmer is provided with the capability for detecting and displaying of the patient's surface ECG via surface electrodes and cable, 118, and ECG detection amplifier 116. The ECG detection amplifier 116 interacts with the programmer control system 102 such that the surface ECG can be displayed on the display device 108. While the preferred embodiment utilizes the intracardiac electrograms and/or markers from the implantable device 10, the present invention may be adapted to assess electrical signals from the surface ECG for the capture verification assessment function.

The medical practitioner interacts with the programmer 100 through a user input device 106, which may for example be a keyboard, a pen, or a voice interface. Through the user input device 106, the medical practitioner may also issue commands to the implantable stimulation device 10 when the implantable stimulation device 10 is in communication with the programmer 100. An optional printer 114 may be used to print the results of the capture verification and pacing threshold determination test at the medical practitioner's request.

The operation of the implantable stimulation device 10 is generally controlled by a control program stored in the memory 14 and executed by the control system 30. This control program usually consists of multiple integrated program modules, with each module bearing responsibility for controlling one or more functions of the implantable stimulation device 10. For example, one program module may control the delivery of stimulating pulses to the heart 24, while another module may control the verification of atrial and/or ventricular capture and pacing threshold determination. In effect, each program module is a control program dedicated to a specific function or a set of functions of the implantable stimulation device 10.

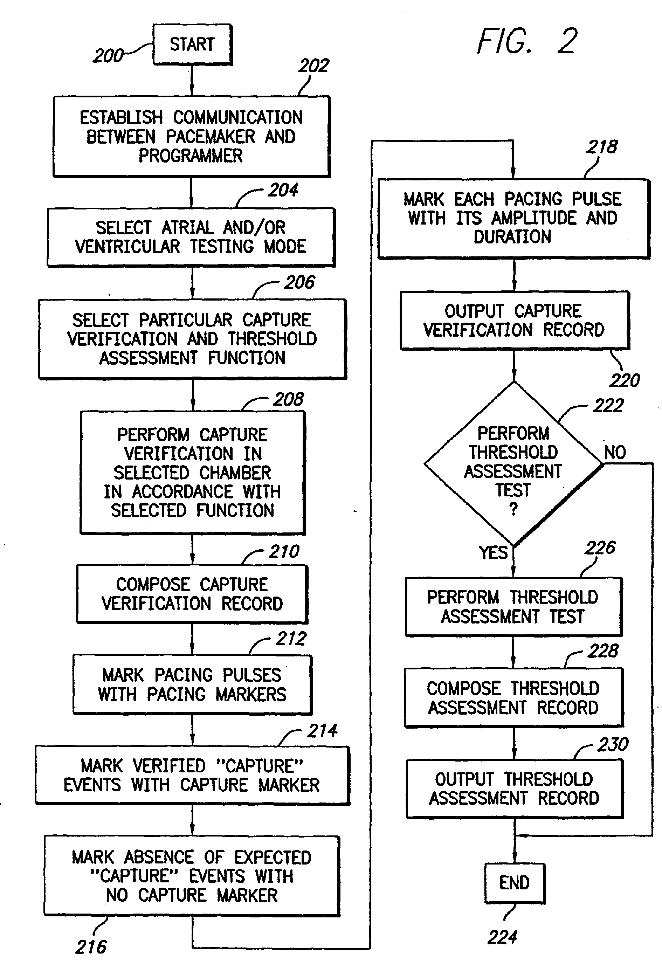

Similarly, the operation of the programmer 100 is generally controlled by a main control program stored in the programmer memory 104 and executed by the programmer control system 102. This main control program also consists of multiple integrated program modules that correspond to various features of the programmer 100. The control program module dedicated to controlling the capture verification and pacing threshold assessment procedure is described below in connection with **FIG. 2**. The control program module of **FIG. 2** automatically interacts with appropriate control program modules of the implantable stimulation device 10 to conduct one or more portions of the procedure.

Referring now to **FIG. 2**, a logic flow diagram representing the control program for controlling the capture verification assessment and pacing threshold determination procedure executed by the programmer control system 102 of **FIG. 1** in accordance with the present invention is described.

After the control program begins at a step 200, the programmer control system 102 establishes communication between the implantable stimulation device 10 and the programmer 100. At a step 204, the medical practitioner selects one of the chambers of the heart 24 in which capture verification and/or pacing threshold assessment is to be performed. Either the atrial or the ventricular chamber may be selected.

At an optional step 206, the medical practitioner may select a particular capture verification assessment and/or threshold assessment function. Depending on their individual configurations, advanced implantable stimulation device and programmer models may be capable of several different capture verification assessment and threshold assessment functions. Thus, it may be advantageous for the medical practitioner to be able to select a capture verification function that is desirable for a particular patient or for a particular implantable stimulation device 10 and programmer 100 configuration. For example, if the programmer 100 has superior IEGM analysis capabilities, it may be advantageous to select a capture verification function that acquires the IEGM through the implantable stimulation device 10 and then utilizes the superior analysis capabilities of the programmer 100 to verify that capture is present. Examples of various superior capture verification functions are disclosed in the U.S. Patent No. 5,417,718 (Kleks et al.); U.S. Patent No. 5,549,652 (McClure et al.); and U.S. Patent No. 4,817,605 (Sholder).

At a step 208, the programmer control system 102 initiates capture verification assessment using a function selected at the step 206. Depending on the particular function selected, the actual capture verification assessment may be performed by the control system 30, by the programmer control system 102, or by both systems 30 and 102 working in conjunction with one another.

At a step 210, the implantable stimulation device control system 102 composes a capture verification record representative of cardiac events in the atrial and ventricular channels, such as P-waves and R-waves, and implantable stimulation device events, such as A-pulses and V-pulses. The cardiac events are identified by the control system 30 from signals received through the atrial and ventricular sense amplifiers 26 and 28, respectively, while the pacing pulses correspond to the trigger signals that are transmitted by the control system 30 to the atrial and ventricular pulse generators 16 and 22, respectively. In one embodiment of the present invention, the capture verification record may be based on an IEGM measured by the control system 30.

Alternatively, the programmer control system 102 may utilize the surface ECG of the patient via the electrodes and cable 118 and the surface ECG input amplifier 116 for the purposes of performing the capture verification assessment function. For example, the programmer control system may store a particular surface ECG waveform morphology consistent with ventricular capture in the memory 104 of the programmer. By performing beat by beat comparison of waveform morphology, the programmer will assess the consistency of capture and generate a capture record for display on the programmer display device 108.

At a step 212, the programmer control system 102 marks A- and/or V- pacing pulses in each of the ventricular and atrial channel portions of the capture verification record with an appropriate PACING PULSE marker. For example, an A-pulse may be marked with a letter "A", while a V-pulse may be marked with a letter "V". Other markers representative of pacing pulses may also be used as a matter of design choice.

At a step 214, the programmer control system 102 marks verified capture events in the capture verification record with a CAPTURE marker. The CAPTURE marker may be a text marker, such as the word "capture" displayed next to a capture event, or it may be a letter "C". Other markers representative of a capture event may also be used as a matter of design choice. At a step 216, the programmer control system 102 places a NO CAPTURE marker in each location on the capture verification record where a capture event is expected but does not occur. The NO CAPTURE marker may be a text marker, such as the words "no capture" displayed next to a capture event, or it may be the letters "NC". Other markers representative of the absence of a capture event may also be used as a matter of design choice.

At an optional step 218, the programmer control system 102 may also mark the pacing pulses with markers representative of their respective amplitude and duration characteristics. These markers provide the medical practitioner with additional information useful for analysis of the capture verification record.

It should be noted that the steps 210 through 218 may alternately be performed by the control system 30 of the implantable stimulation device 10 as a matter of design choice without departing from the spirit of the invention. Thus, the capture verification record may be composed at the implantable stimulation device 10 and then transmitted to the programmer 100 via the communication link 112.

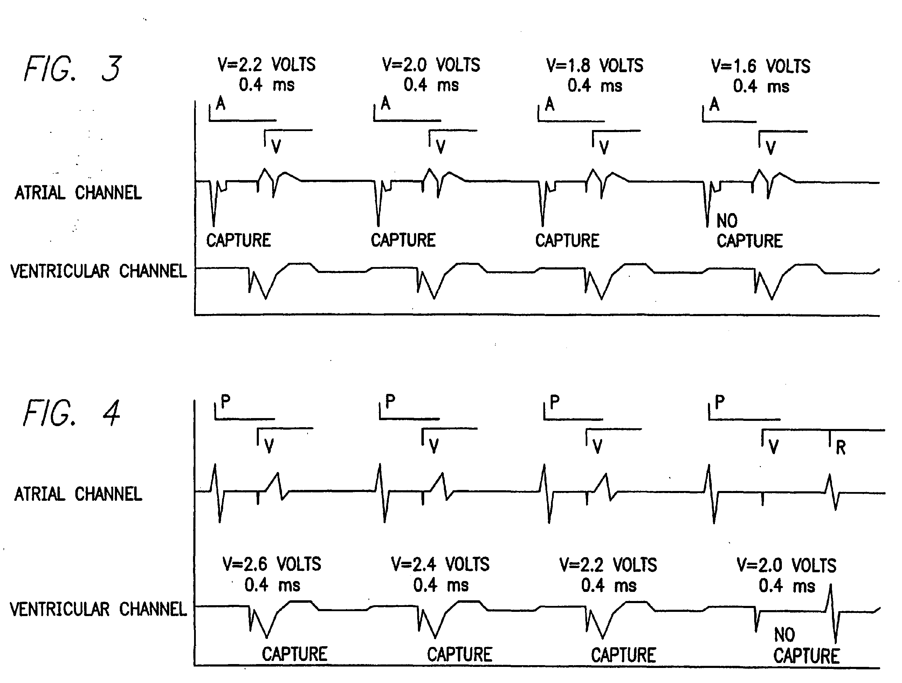

Referring now to **FIG. 3**, an exemplary capture verification and threshold assessment record of an atrial capture test is shown. In the exemplary record, the atrial capture threshold is set to 1.8 volts, and thus a 1.6 volt pacing pulse does not capture. CAPTURE markers are indicated with "capture", and the NO CAPTURE marker is indicated with "no capture". The amplitude and duration of pacing pulses is also indicated. Referring now to **FIG. 4**, an exemplary capture verification and threshold assessment record of a ventricular capture test is shown. In the exemplary record, the ventricular capture threshold is set to 2.2 volts and thus a 2.0 volt pacing pulse does not capture.

Returning now to **FIG. 2**, at a step 220, the programmer control system 102 displays the capture verification record to the medical practitioner on the display device 108. Optionally, the capture verification record may also be printed by the printer 114.

At a test 222, the medical practitioner is prompted by the programmer control system 102 to indicate whether pacing threshold assessment is to be conducted. If the capture verification record shows loss of capture, the medical practitioner will most likely initiate the pacing threshold assessment procedure. If the medical practitioner indicates that pacing capture threshold assessment is to be conducted, the programmer control system 102 proceeds to a step 226. Otherwise, the programmer control system 102 proceeds to a step 224 where the control program ends. Optionally, the pacing capture threshold assessment test may be initiated automatically without input from the medical practitioner.

At the step 226, the programmer control system 102 performs a pacing capture threshold assessment test to determine an appropriate pacing threshold for the implantable stimulation device 10. Examples of automatic pacing threshold assessment tests are disclosed in the U.S. Patent No. 5,417,718 to Kleks et al.; U.S. Patent No. 5,549,652 to McClure et al.; and U.S. Patent No. 4,817,605 to Sholder. At a step 228, the programmer control system 102 composes a pacing threshold assessment record that indicates the atrial and/or ventricular capture thresholds and also indicates the appropriate pacing thresholds for the atrial and/or ventricular channels. During an automatic threshold assessment function the programmer controls the temporary programming of stimulus capture energy and terminates the threshold assessment function as soon as loss of capture is detected.

At a step 230, the programmer control system 102 displays the pacing capture threshold assessment record to the medical practitioner on the display device 108. Optionally, the pacing capture threshold assessment record may also be printed by the printer 114. The programmer control unit 102 then proceeds to the step 224 where the control program operation ends.

The medical practitioner is thus presented with complete visual records of automatic capture verification assessment and automatic capture threshold assessment, so that the records may be analyzed and the proper operation of the implantable stimulation device 10 confirmed without subjecting the patient to intensive time consuming procedures such as analysis of the surface ECG during the threshold determination sequence performed by the medical practitioner.

## Claims

1. A system for automating review of capture verification by a medical practitioner, the system being configured for use with an implantable stimulation device implanted in a patient and a programmer operated by the medical practitioner and configured to remotely communicate with the implantable stimulation device, the system comprising: autocapture means for performing automatic capture verification through the implantable stimulation device to detect a presence of a captured cardiac event and an absence of a captured cardiac event when the captured cardiac event is expected; control means for generating a visual representation; and display means for displaying the visual representation to the medical practitioner, to permit the medical practitioner to examine and analyse the performance of the automatic capture verification; **characterised in that** the visual representation is a representation of the presence and absence of the captured cardiac event and the control means further comprises means for marking the captured cardiac event in the visual representation with a visual marker representative of capture and means for marking absence of the captured cardiac event with a visual marker representative of absence of capture in a location in the visual representation where the captured cardiac event was expected to occur.

2. The system of Claim 1, further comprising: first selection means, operatively coupled to the autocapture means, for selecting one of an atrial and a ventricular chamber of the patient's heart at which the capture verification is performed.

3. The system of Claim 1, wherein the autocapture means comprises a plurality of unique means for automatic capture verification; and the system further comprises second selection means, operatively coupled to the autocapture means, for selecting a particular one of the unique automatic capture verification means for performing the automatic capture verification.

4. The system of claim 1, wherein:
the autocapture means further comprises means for detecting a plurality of additional cardiac events occurring during the automatic capture verification, and
the control means further comprises means for identifying the plurality of additional cardiac events in the visual representation.

5. The system of claim 4, wherein the plural additional cardiac events comprise physiological atrial and ventricular events.

6. The system of claim 1, wherein the control means further comprises:
means for identifying in the visual representation a plurality of pacing events occurring during the automatic capture verification.

7. The system of claim 6, wherein the plural pacing events comprise atrial and ventricular pacing pulses.

8. The system of claim 7, wherein:
each of the plural pacing events comprise amplitude and duration characteristics; and
the control system further comprises means for recording, in the visual representation, the amplitude and duration characteristics for the each pacing event of the plurality of pacing events.

9. The system of Claim 1, further comprising means for automatically assessing a pacing threshold value of the implantable stimulation device; means for adding a suitable safety margin to the pacing threshold value to determine recommended pacing amplitude and pulse width; and means for displaying the recommended pacing amplitude and pulse width.

10. The system of Claim 1, further comprising third selection means operable for selectively activating the autothreshold means from the programmer.

11. The system of Claim 1, wherein the control means further comprises means for generating an additional visual representation of the pacing threshold assessment; and the additional visual representation is displayed to the medical practitioner on the display means, to permit the medical practitioner to examine and analyse the performance of the automatic pacing threshold assessment.

12. The system of any preceding claim, further comprising: printing means operatively coupled to the control means for generating a printed copy of the visual representation and/or the additional visual representation.

## Patentansprüche

1. System zum Automatisieren der Nachprüfung der Festsetzung des Einfangs durch einen medizinischen Praktiker, wobei das System zur Verwendung mit einer implantierbaren Stimulationseinrichtung, die in einem Patienten implantiert ist, und einer Programmiereinrichtung konfiguriert ist, die von dem medizinischen Praktiker betätigt wird und konfiguriert ist, um mit der implantierbaren Stimulationseinrichtung über Distanz zu kommunizieren, wobei das System aufweist automatische Einfang-Festsetzmittel zur Durchführung der automatischen Festsetzung des Einfangs durch die implantierbare Stimulationseinrichtung, um einer Anwesenheit eines eingefangenen Herzereignisses und einer Abwesenheit eines eingefangenen Herzereignisses zu detektieren, wenn das eingefangene Herzereignis erwartet wird; Steuermittel zur Erzeugung einer visuelle Darstellung und Anzeigemittel, um die visuelle Darstellung dem medizinischen Praktiker anzuzeigen, um es dem medizinischen Praktiker zu ermöglichen, die Arbeitsweise der automatischen Festsetzung des Einfangs zu überprüfen und zu analysieren, **dadurch gekennzeichnet, dass** die visuelle Darstellung eine Darstellung der Anwesenheit oder Abwesenheit des eingefangenen Herzereignisses ist, und dass die Steuermittel ferner Mittel, um das eingefangene Herzereignis in der visuellen Darstellung mit einer visuellen Marke, die den Einfang repräsentiert, zu markieren, und Mittel umfasst, um die Abwesenheit des eingefangenen Herzereignisses mit einer visuellen Markierung, die die Abwesenheit des Einfangs darstellt, an einer Stelle der visuellen Darstellung zu markieren, an der das Auftreten des erwarteten Herzereignisses zu erwarten war.

2. System nach Anspruch 1 ferner umfassend erste Auswahlmittel, die wirksam mit den automatischen Feststellungsmitteln gekoppelt sind, um eine von einer atrialen und einer ventrikulären Kammer des Herzens des Patienten auszuwählen, an der die Festsetzung des Einfangs durchgeführt wird.

3. System nach Anspruch 1, worin die automatischen Einfangmittel eine Vielzahl von einzigartigen Mitteln zur automatischen Festsetzung des Einfangs aufweisen, und dass das System ferner zweite Auswahlmittel umfasst, die wirksam mit den automatischen Einfangmitteln gekoppelt sind, um ein spezielles der einzigartigen, automatischen Einfang-Festsetzungsmittel auszuwählen, um die automatische Festsetzung des Einfangs durchzuführen.

4. System nach Anspruch 1, worin die automatischen Einfangmittel ferner Mittel umfassen, um eine Vielzahl von zusätzlichen Herzereignissen zu detektieren, die während der automatischen Festsetzung des Einfangs auftreten, und worin die Steuermittel ferner Mittel umfassen, um die Vielzahl der zusätzlichen Herzereignisse in der visuellen Darstellung zu identifizieren.

5. System nach Anspruch 4, worin die Vielzahl der zusätzlichen Herzereignisse physiologische, atriale und ventrikuläre Ereignisse umfassen.

6. System nach Anspruch 1, worin die Steuermittel ferner umfassen: Mittel zur Identifizierung der visuellen Darstellung einer Vielzahl von Stimulationsereignissen, die während der automatischen Festsetzung des Einfangs auftreten.

7. System nach Anspruch 6, worin die Vielzahl der Stimulationsereignisse atriale und ventrikuläre Stimulationspulse umfassen.

8. System nach Anspruch 7, worin jedes der Vielzahl der Stimulationsereignisse Amplituden-und Dauer- Charakteristiken umfasst, und worin das Steuersystem ferner Mittel umfasst, um in der visuellen Darstellung die Amplituden- und Dauer- Charakteristiken von jedem Stimulationsereignis in der Vielzahl der Stimulationsereignisse aufzuzeichnen.

9. System nach Anspruch 1, ferner umfassend Mittel zur automatischen Festsetzung eines Stimulationsschwellenwertes der implantierbaren Stimulationseinrichtung, Mittel zum Hinzufügen eines geeigneten Sicherheitsspielraums zu dem Stimulationsschwellenwert, um eine empfohlene Stimulationsamplitude und Pulsbreite zu bestimmen, und Mittel zur Anzeige der empfohlenen Stimulationsamplitude und- Pulsbreite.

10. System nach Anspruch 1, ferner umfassend dritte Auswahlmittel, die betreibbar sind, um die automatischen Schwellenwertmittel von der Programmiereinrichtung her selektiv zu aktivieren.

11. System nach Anspruch 10, worin die Steuermittel ferner Mittel umfassen, um eine zusätzliche visuelle Darstellung der Stimulationsschwellenwert-Festsetzung zu erzeugen, und worin die zusätzliche visuelle Darstellung dem medizinischen Praktiker auf der Anzeigeeinrichtung angezeigt wird, um es dem medizinischen Praktiker zu ermöglichen, die Arbeitsweise der automatischen Stimulationsschwellenwert-Festsetzung zu überprüfen und zu analysieren.

12. System nach einem der vorhergehenden Ansprüche ferner umfassend Druckermittel, die wirksam mit den Kontrollmitteln gekoppelt sind, um eine gedruckte Kopie der visuellen Darstellung und/ oder der zusätzlichen visuellen Darstellung zu erzeugen.

## Revendications

1. Système destiné à automatiser la révision de la vérification de capture par un praticien, le système étant configuré pour une utilisation avec un dispositif de stimulation implantable implanté dans un patient et un sélecteur de programme géré par le praticien et configuré pour communiquer à distance avec le dispositif de stimulation implantable, le système comprenant : des moyens de capture automatique destinés à effectuer une vérification de capture automatique par l'intermédiaire du dispositif de stimulation implantable afin de détecter la présence d'un événement cardiaque capturé et l'absence d'un événement cardiaque capturé lorsque l'événement cardiaque capturé est attendu ; des moyens de contrôle destinés à générer une représentation visuelle ; et des moyens d'affichage destinés à afficher la représentation visuelle à l'attention du praticien, afin de permettre au praticien d'examiner et d'analyser les performances de la vérification de capture automatique ; **caractérisé en ce que** la représentation visuelle est une représentation de la présence et de l'absence d'un événement cardiaque capturé et les moyens de contrôle comprennent en outre des moyens destinés à marquer l'événement cardiaque capturé sur la représentation visuelle au moyen d'un marqueur visuel représentatif de la capture et des moyens destinés à marquer l'absence de l'événement cardiaque capturé au moyen d'un marqueur visuel représentatif de l'absence de la capture au niveau d'une localisation sur la représentation visuelle où il était attendu que l'événement cardiaque capturé se produise.

2. Système selon la revendication 1, comprenant en outre : des premiers moyens de sélection, couplés de manière opérationnelle aux moyens de capture automatique, destinés à sélectionner l'une des chambres atriale et ventriculaire du coeur du patient au niveau de laquelle la vérification de capture est effectuée.

3. Système selon la revendication 1; dans lequel les moyens de capture automatique comprennent une pluralité de moyens uniques pour une vérification de capture automatique ; et le système comprend en outre des deuxièmes moyens de sélection, couplés de manière opérationnelle aux moyens de capture automatique, destinés à sélectionner des moyens particuliers parmi les moyens uniques de vérification de capture automatique afin d'effectuer la vérification de capture automatique.

4. Système selon la revendication 1, dans lequel :
les moyens de capture automatique comprennent en outre des moyens destinés à détecter une pluralité d'événements cardiaques supplémentaires se produisant au cours de la vérification de capture automatique, et
les moyens de contrôle comprennent en outre des moyens destinés à identifier la pluralité d'événements cardiaques supplémentaires sur la représentation visuelle.

5. Système selon la revendication 4, dans lequel la pluralité d'événements cardiaques supplémentaires comprend des événements atriaux et ventriculaires physiologiques.

6. Système selon la revendication 1, dans lequel les moyens de contrôle comprennent en outre :
des moyens destinés à identifier sur la représentation visuelle une pluralité d'événements de stimulation du coeur se produisant au cours de la vérification de capture automatique.

7. Système selon la revendication 6, dans lequel la pluralité d'événements de stimulation du coeur comprend des impulsions de stimulation du coeur atriale et ventriculaire.

8. Système selon la revendication 7, dans lequel :
chacun des événements de la pluralité d'événements de stimulation du coeur comprend des caractéristiques de l'amplitude et de la durée; et
le système de contrôle comprend en outre des moyens destinés à enregistrer, sur la représentation visuelle, les caractéristiques de l'amplitude et de la durée pour chaque événement de stimulation du coeur de la pluralité d'événements de stimulation du coeur.

9. Système selon la revendication 1, comprenant en outre des moyens destinés à estimer de manière automatique une valeur de seuil de stimulation du coeur du dispositif de stimulation implantable ; des moyens destinés à ajouter une marge de sécurité appropriée à la valeur de seuil de stimulation du coeur afin de déterminer l'amplitude et la longueur d'impulsion de stimulation du coeur recommandées ; et des moyens destinés à afficher l'amplitude et la longueur d'impulsion de stimulation du coeur recommandées.

10. Système selon la revendication 1, comprenant en outre des troisièmes moyens de sélection utilisables afin d'activer de manière sélective les moyens de seuil automatique à partir du sélecteur de programme.,

11. Système selon la revendication 1, dans lequel les moyens de contrôle comprennent en outre des moyens destinés à générer une représentation visuelle supplémentaire de l'estimation de seuil de stimulation du coeur ; et la représentation visuelle supplémentaire est affichée à l'attention du praticien sur les moyens d'affichage, afin de permettre au praticien d'examiner et d'analyser les performances de l'estimation de seuil de stimulation du coeur automatique.

12. Système selon l'une quelconque des revendications précédentes, comprenant en outre: des moyens d'impression couplés de manière fonctionnelle aux moyens de contrôle destinés à générer une copie imprimée de la représentation visuelle et/ou de la représentation visuelle supplémentaire.
